# EUROPÄISCHE PATENTSCHRIFT

(11) **EP 1 094 796 B1**
(45) Veröffentlichungstag und Bekanntmachung des Hinweises auf die Patenterteilung: **15.10.2003**
(21) Anmeldenummer: 99932827.1
(22) Anmeldetag: 06.07.1999
(51) Int. Cl.: A61K 9/70, A61K 31/19

(54) **TOPISCHES PFLASTER MIT NICHTSTEROIDALEN ANTIRHEUMATIKA MIT SÄUREGRUPPE**
TOPICAL PLASTER WITH NON-STEROIDAL ANTIRHEUMATIC AGENTS WITH AN ACID GROUP
EMPLATRE TOPIQUE COMPORTANT DES AGENTS ANTIRHUMATISMAUX NON STEROIDIENS A GROUPE ACIDE

(30) Priorität: 09.07.1998 DE 19830649
(43) Veröffentlichungstag der Anmeldung: 02.05.2001
(73) Patentinhaber: LTS Lohmann Therapie-Systeme AG, 56626 Andernach (DE)
(72) Erfinder: MÜLLER, Walter, D-56564 Neuwied (DE)
(74) Vertreter: Flaccus, Rolf-Dieter, Dr.
(86) Internationale Anmeldenummer: EP9904686
(87) Internationale Veröffentlichungsnummer: WO00002539

(56) Entgegenhaltungen:
- EP-A- 0 319 988
- DE-C- 19 706 824
- PATENT ABSTRACTS OF JAPAN vol. 017, no. 704 (C-1146), 22. Dezember 1993 (1993-12-22) & JP 05 238931 A (NITTO DENKO), 17. September 1993 (1993-09-17) -& DATABASE WPI Derwent Publications Ltd., London, GB; AN 1993-331354 XP002117847 & JP 05 238931 A (NITTO DENKO), 22. Dezember 1993 (1993-12-22)

## Beschreibung

Eine große Gruppe der sogenannten nichtsteroidalen Antirheumatika sind Wirkstoffe, die als Derivate der Essigsäure und der Propionsäure aufzufassen sind.

Vertreter der Essigsäurederivate sind dabei ohne Anspruch auf Vollständigkeit Indomethacin, Acemetacin, Tolmetin, Diclofenac und Lonazolac; Vertreter der Propionsäurederivate (Profene) Ibuprofen, Flurbiprofen, Fenoprofen, Ketoprofen, Naproxen und Tiaprofen.

Die freie Carboxylgruppe ist für die Wirkung dieser Substanzklasse insofern von Bedeutung, als sie zu einer Anreicherung der Wirkstoffe in entzündlichen Geweben mit einem erniedrigten pH-Wert führt.
Für die perorale Verabreichung werden allerdings wegen der besseren Löslichkeit im wäßrigen Milieu oftmals nicht die freien Säuren, sondern die Salze eingesetzt. Für die topische Verabreichung sind allerdings die freien Säuren besser geeignet, da elektrisch neutrale Substanzen das Stratum Corneum der menschlichen Haut besser zu durchdringen vermögen als elektrisch geladene Salze.

Bei allen oben genannten Substanzen ist als Nebenwirkung das Auftreten von Magenbeschwerden und Blutungen im Gastrointestinalbereich beschrieben. Es ist deshalb von Vorteil, bei lokalen Beschwerden diese Substanzen nicht systemisch, sondern lokal zu verabreichen.
Solche Beschwerden sind z.B. entzündlich-rheumatische Erkrankungen der Gelenke und der Wirbelsäule, Schwellungen und Entzündungen der Weichteile in der Nähe von Gelenken, Schultersteife, Kreuzschmerzen, Hexenschuß sowie Sport- und Unfallverletzungen.

Für die lokale Verabreichung können Gele, Salben oder selbstklebende Pflastersysteme genommen werden. Die selbstklebenden Pflastersysteme haben dabei gegenüber den Salben und Gelen den Vorteil, daß die Kleidung nicht kontaminiert wird und die Pflaster bei entsprechender Auslegung nur einmal alle 1-2 Tage appliziert werden müssen.

Solche Pflaster zur topischen Applikation am Wirkort bestehen im allgemeinen aus einer wirkstoffhaltigen, selbstklebenden sogenannten Matrixschicht, einer oftmals textilen Rückschicht und einer vor Gebrauch zu entfernende Schutzschicht für die Matrix.

Bedingt durch die nur topische Wirkung am Applikationsort haben solche Pflaster eine Größe, die bei etwa 70 cm² beginnt und bis zu etwa 250 cm² reicht. Dies bedeutet, daß die physikalischen Eigenschaften der Rückschicht eine wichtige Rolle.bei den Trageeigenschaften des Pflasters spielen. Speziell bei der Anwendung im Gelenkbereich zeigt es sich, daß die Rückschicht zumindest in einer Richtung elastisch sein muß, um einerseits in diesem Bereich genügend gut zu kleben und andererseits den Bewegungsspielraum nicht zu sehr einzuengen. Folienartige Materialien sind entweder nicht elastisch, oder wenn sie elastisch sind, aus Materialien hergestellt, die nicht inert gegenüber den Inhaltsstoffen der Pflastermatrix sind.
Zudem ist bei Folien die Wasserdampfdurchlässigkeit in Anhängigkeit von den ausgewählten Materialien oft ein Problem, da Okklusion und das damit verbundene Schwitzen die Klebeeigenschaften erheblich beeinträchtigen können.

Auch textile Materialien sind nicht ohne Probleme, da Materialien wie Baumwolle oder Polyurethane dazu neigen, Wirkstoffe oder diffusible Hilfsstoffe zu binden bzw. aufzunehmen. Speziell Polyurethane neigen dazu, ihre physikalischen Eigenschaften in unzulässiger Weise zu verändern.

Auch an den Kleber sind spezielle Anforderungen zu stellen. Seine wichtigste Funktion ist es, das System für die beabsichtigte Tragezeit sicher auf der Haut zu verankern, ohne beim Abnehmen zu Schmerzen oder zu Hautabrissen zu führen. Er sollte nicht okklusiv wirken, da die Hautverträglichkeit mit zunehmender Okklusion abnimmt. Da er in innigem Kontakt mit dem Wirkstoff steht, muß er genügend inert diesem gegenüber sein, um ein zumindest für zwei Jahre stabiles Pflaster zu erhalten. Die Zusammensetzung des Klebers muß dabei auf die jeweilige chemische Zusammensetzung der Wirkund Hilfsstoffe in geeigneter Weise zugeschnitten sein. Nicht zuletzt muß der Kleber über eine geeignete Löslichkeit für die Wirkstoffe verfügen. Da die Permeationsrate wesentlich von der thermodynamischen Aktivität abhängt, ist eine Wirkstoffkonzentration möglichst nahe der Sättigungslöslichkeit anzustreben. Im allgemeinen ist wegen der doch relativ hohen abzugebenden Wirkstoffmenge eine Löslichkeit von mindestens 5 % (g/g), und aus Gründen der Wirkstoffersparnis von nicht mehr als 30 %, besser nicht mehr als 15 % (g/g), anzustreben.

Allen diesen Ansprüchen werden dabei am ehesten Polyacrylatkleber gerecht. Diese Kleber werden durch die radikalischen Polymerisation von Acryl- bzw. Methacrylsäure und ihren Derivaten hergestellt. Zusätzlich mögliche Monomere sind Vinylverbindungen wie z.B. Vinylacetat oder Maleinsäure.

Neben den mehr technischen Aspekten spielt bei topischen Systemen die Hautverträglichkeit eine große Rolle. Während systemisch wirksame transdermale therapeutische Systeme (TTS) auf wechselnde Hautareale appliziert werden, ist bei topischen Pflastern der Applikationsort durch das Leiden vorgegeben. Dies bedeutet, daß für solche Pflaster nur gut hautverträgliche Inhaltsstoffe für die Matrix genommen werden können und darüber hinaus das Klebeverhalten so ausgelegt sein muß, daß einerseits das Pflaster über die beabsichtigte Applikationszeit zuverlässig klebt und andererseits beim Abnehmen des Pflasters keine zu große mechanische Reizung der Haut erfolgt.

Selbstverständlich müssen die Pflaster in der Lage sein, genügend Wirkstoff abzugeben, um in den unter den Pflastern liegenden Geweben, das heißt dem Wirkort, ausreichend hohe Gewebsspiegel zu erreichen.

Ebenfalls selbstverständlich ist die Forderung nach einer genügenden Stabilität der Arzneiform bezüglich des Wirkstoffgehalts, der Wirkstoffabgabe und dem Klebeverhalten.

Zusammenfassend sollen topische Pflaster im wesentlichen folgende Anforderungen möglichst optimal erfüllen:
- genügend hohe Permeationsrate zum Erzielen therapeutisch effektiver Gewebespiegel an der Applikationsstelle,
- gute Hautverträglichkeit bei Mehrfachapplikation an der gleichen Stelle,
- gutes, aber nicht zu festes Kleben und kein Stripping beim Entfernen,
- Elastizität in mindestens einer Richtung, um Anwendung im Gelenkbereich zu ermöglichen,
- Stabilität über mindestens 2 Jahre,
- einfache und kostengünstige Herstellung.

Der vorliegenden Erfindung lag die Aufgabe zugrunde, ein topisches Pflaster mit nichtsteroidalen Antirheumatika mit freien Carboxylgruppen bereitzustellen, welches die vorgenannten Anforderungen erfüllt.
Diese Aufgabe wurde in überraschender Weise für die Wirkstoffgruppe der nichtsteroidalen Antirheumatika mit freien Carboxylgruppen durch ein Pflaster gemäß den Merkmalen des Hauptanspruches gelöst.

In der patentliteratur sind topische Pflaster beschrieben, die auch nichtsteroidale Wirkstoffe umfassen. Nicht berücksichtigt sind dabei Pflaster auf der Basis von Hydrogelen, da diese wegen ihrer geringen Klebkraft nur eingeschränkt ohne zusätzliche Fixierverbände anzuwenden sind.

In der GB 2 273 044 sind z.B. Pflaster beschrieben, die auch Ketoprofen als wirksamen Bestandteil umfassen. In ihnen ist der Wirkstoff in der Matrix kombiniert mit die Permeation durch die Haut verbessernden Substanzen aus der Gruppe der Fettsäureester, Polyoxyethylenderivate, Glyceride, Fettsäureester des Propylenglykols und Pyrrolidonderivate. Der Kleber kann dabei auch aus der Gruppe der Polyacrylatkleber stammen. Nichts ausgesagt wird über die physikalischen Eigenschaften der Rückschicht. Als Material für textile Rückschichten wird Baumwolle erwähnt, die jedoch einen Großteil des in der Matrix enthaltenden Wirkstoffes bindet und dadurch die Wirkstoffabgabe negativ beeinflußt.
Saure funktionelle Gruppen im Polymer sowie die Verwendung eines carboxylgruppenhaltigen Weichmachers bzw. Permeationsverbesserers werden nicht beschrieben.

Ein topisches Pflaster mit dem Wirkstoff Ketoprofen ist beschrieben in der DE-OS 195 27 306. Dieses Pflaster ist gekennzeichnet durch eine mehrschichtige Matrix, wobei die einzelnen Schichten über ein verschieden hohes Wasseraufnahmevermögen verfügen.

In der DE-OS 197 06 824 wird ein topisches Pflaster mit Ketoprofen/Tiaprofensäure als Wirkstoff beschrieben, das aus einer Rückschicht, einer selbstklebenden, wirkstoffhaltigen, mindestens zweischichtigen Matrixschicht auf Basis eines mit mehrwertigen Metallionen vernetzten Polyacrylatklebers und einer Schutzfolie besteht, wobei die wirkstoffhaltige Matrix Ölsäure enthält und die Rückschicht aus einem querelastischen Gewebe aus Viskose besteht. Bei diesen Pflastern ist die hautseitige, dünnere Matrixschicht weniger stark vernetzt als die hautferne Matrixschicht, wodurch dieses Pflaster gute Klebeigenschaften aufweist und der "Kalte Fluß" während der Lagerung verhindert wird. Allerdings ist die Herstellung von Pflastern, die mindestens zwei Matrixschichten aufweisenden, relativ aufwendig.

In der US 5,702,720 ist ein Pflaster mit Flurbiprofen als Wirkstoff beschrieben. Die Matrix dieses Pflasters besteht auch aus einem Polyacrylatkleber, der Polyvinylpyrrolidon als zusätzliche Komponente enthält. Polyvinylpyrrolidon ist dabei als nachteilig zu betrachten, da dieses Polymer eine starke Wechselwirkung mit Carboxylgruppen und phenolischen OH-Gruppen zeigt. Es verbessert zwar die Haftung auf der Haut, allerdings um den Preis einer geringeren Wirkstoffabgabe bzw. einer höheren notwendigen Wirkstoffmenge im Pflaster.

In der WO 95/31193 ist ein Pflaster mit Ibuprofen als Wirksubstanz beschrieben. Hier besteht die Matrix aus zwei unterschiedlichen Polyacrylatpolymeren und - neben dem Wirkstoff - zusätzlich aus Diethylphthalat. Diethylphthalat ist dabei nicht als toxikologisch unbedenklich zu betrachten, da es in erheblichen Mengen die Haut zu durchdringen vermag. Ein saurer Weichmacher in Verbindung mit sauren funktionellen Gruppen in der Matrix und einem nichtsteroidalen Antirheumatikum mit freien Carboxylgruppen sind nicht beschrieben.

Keines der im Stand der Technik erwähnten Pflaster enthält alle für ein topisches Pflaster notwendigen Elemente in optimierter Form.

Es war überraschend, daß die Kombination eines Wirkstoffes mit einer freien Carboxylgruppe mit einem vernetzten Acrylatkleber mit freien Carboxylgruppen aus der einpolymerisierten Acryl- oder Methacrylsäure und einer Fettsäure als Weichmacher und Permeationsverbesserer eine Matrix ergibt, die bezüglich ihrer physikalischen Eigenschaften in optimaler Weise allen Anforderungen gerecht wird.

Die Vernetzung des Acrylatklebers erfolgt mit mehrwertigen Metallkationen, bevorzugt mit Aluminium. Die Aluminiumionen werden dabei der Kleberlösung als Aluminiumacetylacetonat zugesetzt. Der organische Anteil der Verbindung wird bei dem Trocknen des Klebers zusammen mit den Lösemitteln entfernt; die Carboxylgruppen des Klebers bilden nun die Gegenionen der Aluminiumkationen. Die dadurch resultierende Vernetzung ist als reversibel zu betrachten. Offensichtlich tritt sowohl der saure Wirkstoff als auch der saure Weichmacher ebenfalls in Wechselwirkung mit den Aluminiumionen und verleiht dadurch der Matrix ein gutes Klebeverhalten, ohne daß die Matrix zu weich wird und dadurch zu sogenanntem "kalten Fluß" neigt.
Dieser kalte Fluß stellt einerseits ein Stabilitätsproblem dar, andererseits macht er sich störend dadurch bemerkbar, daß nach der Abnahme des Pflasters von der Haut Kleberränder auf der Haut verbleiben können.

Es ist ebenfalls davon auszugehen, daß die anwesende Fettsäure solche Stellen im Polymer blockiert, die auch mit dem sauren Wirkstoff in Wechselwirkung treten können und damit Abgabeeigenschaften des sauren Wirkstoffs beeinflussen können.
Ein weiterer Vorteil ist, daß durch die Anwesenheit der Dissoziation der Wirkstoffsäure zugunsten der neutralen Wirkstoffsäure zurückgedrängt und dadurch die die Haut besser permeierende Neutralform des Wirkstoffs begünstigt wird.

Insgesamt ist es also eine Vielzahl von miteinander in Wechselwirkung stehenden Einflüssen, die in ihrer Kombination der Matrix optimale physikalische Eigenschaften verleihen.

Die Rückschicht des Pflasters besteht aus einem zumindest in einer Richtung elastischen Polyestergewebe oder Polyestergewirke oder aus einem elastischen geschlossenzelligen Schaum. Die Polyestergewebe oder Gewirke erhalten ihre Elastizität durch die Elastizität der verwendeten Polyestergarne. Sie unterscheiden sich dadurch von leicht elastischen Polyestervliesen. Solche Vliese sind erhältlich, haben aber den Nachteil, daß sie nur in sehr dünner Form ausreichend elastisch sind und dann die selbstklebende Matrix nicht mehr ausreichend abdecken und vor dem Verkleben mit dem Packstoffmaterial bzw. den Kleidern beim Tragen schützen. Polyestergewebe bzw. Polyestergewirke haben selbst in sehr dicken Ausführungen (ca. 150 g/m²) eine für die Anwendung als textile Rückschichten von Pflastersystemen ausreichende Dehnbarkeit. Der Hauptvorteil in der Verwendung von Polyester liegt allerdings darin, daß dieser von allen für solche Gewebe bzw. Gewirke denkbaren Materialien am inertesten gegenüber diffusiblen Inhaltsstoffen der Matrix ist. Er zeichnet sich dadurch vor allem gegenüber Materialien wie Baumwolle, Viskose, Polyamiden oder Polyvinylacetaten aus.
Selbst bei 3-jähriger Lagerung bleibt die Abgaberate eines Pflasters, wie am Beispiel Ibuprofen und Ketoprofen belegt, bei Verwendung einer Rückschicht aus Polyester unverändert (siehe Tabelle 1). Dies zeigt an, daß keinerlei Wirkstoff durch dieses Material aufgenommen wird.

Auch der Wirkstoffgehalt selbst ist über diese Zeit selbst bei Lagerung unter erhöhter Temperatur konstant. Abbauprodukte werden nicht beobachtet. Dadurch wird die ausgezeichnete Stabilität solcher Pflaster zusätzlich belegt.

In vitro-Permeationsversuche an Humanepidermis belegen, daß auch die Wirkstoffabgabe an die Haut ausreichend hoch ist. Beispielhaft aufgezeigt sei dies an Permeationsversuchen mit Pflastern, die gemäß Beispiel 2 und 3 hergestellt wurden.

Die Permeationsrate kann noch gesteigert werden durch die Verwendung einer Rückschicht aus einem geschlossenzelligen Schaum auf Basis von Polyethylen, Polypropylen, Polyvinylchlorid oder einem Copolymeren aus Ethylen und Vinylacetat. Die Ursache ist eine Erhöhung der Okklusion, die sich generell steigernd auf Permeationsraten auswirkt. Bei Verwendung solcher Schäume als Rückschicht erreicht man zwar nicht die hohe Elastizität und den gleichen Tragekomfort wie bei Verwendung von Polyestergeweben, hat allerdings den Vorteil der höheren Wirksamkeit aufgrund der höheren Abgaberaten des Pflasters für den Wirkstoff.

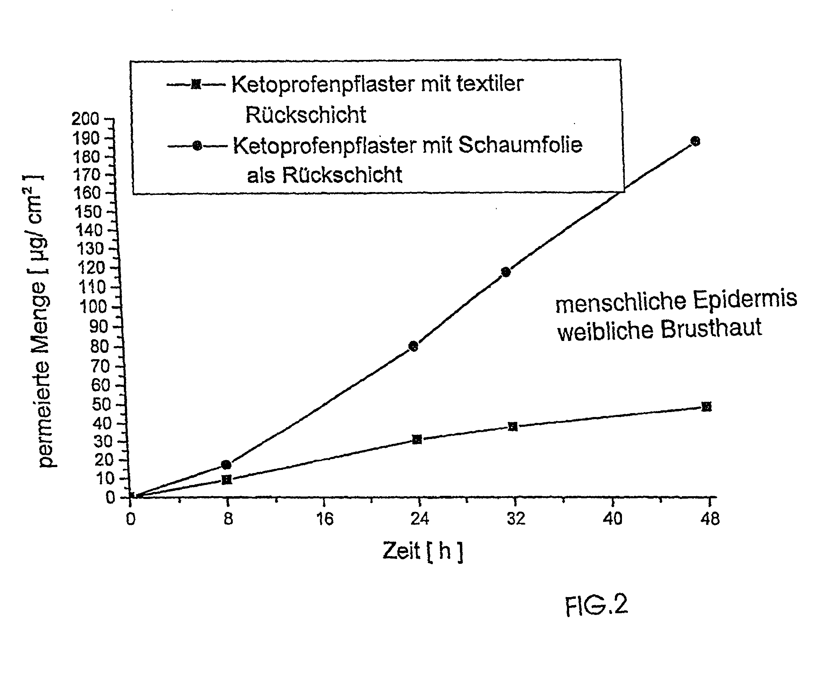

In FIG.2 ist die höhere Permeationsrate anhand einer vergleichenden Permeationsstudie unter Verwendung von menschlicher Epidermis beispielhaft für den Wirkstoff Ketoprofen belegt.

Die Herstellung von Pflastern im Sinne der Erfindung ist dargestellt durch die Beispiele 1-3. Diese Herstellungsweise kann übernommen werden für alle nichtsteroidalen Wirkstoffe mit sauren Gruppen, wobei allerdings für jeden Wirkstoff individuell die geeignete Konzentration gefunden werden muß .

### BEISPIEL 1: Pflaster mit Ketoprofen als Wirkstoff

Zu 500 g Durotak 387-2251 mit einem Feststoffgehalt von 48 Gew.% werden 58 g Ölsäure und 26 g Ketoprofen gegeben und solange gerührt, bis alles Ketoprofen in Lösung gegangen ist.

Anschließend werden 90 g einer 4 %igen (g/g) Lösung von Aluminiumacetylacetonat zugegeben und die Lösung durch Rühren homogenisiert.

Danach wird zur Herstellung der Matrixschicht die Lösung auf eine silikonisierte Folie beschichtet und die Lösemittel durch 20-minütiges Trocknen bei 50 °C entfernt. Die Beschichtungsdicke wird so gewählt, daß der getrocknete Matrixfilm ein Beschichtungsgewicht von 80 g/m² aufweist.

Die getrocknete Matrixschicht wird nun mit einem in zwei Richtungen elastischen Polyestergewebe kaschiert; aus dem dadurch entstandenen Gesamtlaminat werden die fertigen Pflaster ausgestanzt.

### BEISPIEL 2:

Zu 500 g Durotak 387-2251 mit einem Feststoffgehalt von 48 Gew.% werden 58 g Ölsäure und 30 g Flurbiprofen gegeben und solange gerührt, bis alles Flurbiprofen in Lösung gegangen ist.

Anschließend werden 90 g einer 4 %igen Lösung von Aluminiumacetylacetonat zugegeben und die Lösung durch Rühren homogenisiert.

Danach wird zur Herstellung der Matrixschicht die Lösung auf eine silikonisierte Folie beschichtet und die Lösemittel durch 20-minütiges Trocknen bei 50 °C entfernt. Die Beschichtungsdicke wird so gewählt, daß der getrocknete Matrixfilm ein Beschichtungsgewicht von 80 g/m² aufweist.

Die getrocknete Matrixschicht wird nun mit einem in zwei Richtungen elastischen Polyestergewebe kaschiert; aus dem dadurch entstandenen Gesamtlaminat werden die fertigen Pflaster ausgestanzt.

### BEISPIEL 3: Pflaster mit Ibuprofen als Wirkstoff

Zu 500 g Durotak 387-2251 mit einem Feststoffgehalt von 48 Gew.% werden 58 g Ölsäure und 41 g Ibuprofen gegeben und solange gerührt, bis alles Ibuprofen in Lösung gegangen ist.

Anschließend werden 90 g einer 4 %igen Lösung von Aluminiumacetylacetonat zugegeben und die Lösung durch Rühren homogenisiert.

Danach wird zur Herstellung der Matrixschicht die Lösung auf eine silikonisierte Folie beschichtet und die Lösemittel durch 20-minütiges Trocknen bei 50 °C entfernt. Die Beschichtungsdicke wird so gewählt, daß der getrocknete Matrixfilm ein Beschichtungsgewicht von 150 g/m² aufweist.

Die getrocknete Matrixschicht wird nun mit einem in zwei Richtungen elastischen Polyestergewebe kaschiert; aus dem dadurch entstandenen Gesamtlaminat werden die fertigen Pflaster ausgestanzt.

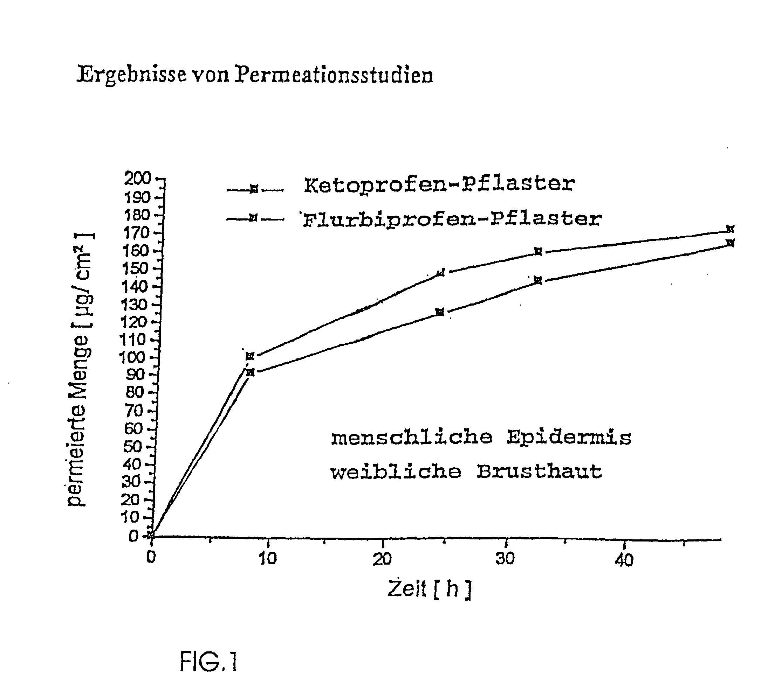

Die in Figur 1 dargestellten Permeationsergebnisse wurden durch in-vitro Permeationsstudien an menschlicher Epidermis unter Verwendung der allgemein bekannten Franz-Diffusionszelle ermittelt.

## Patentansprüche

1. Topisches Pflaster mit einem nichtsteroidalen, über eine freie Carboxylgruppe verfügenden Antirheumatikum als Wirkstoff, bestehend aus
- einer gegenüber dem Wirkstoff inerten Rückschicht, welche aus einem zumindest in eine Richtung elastischen Material besteht,
- einer selbstklebenden, wirkstoffhaltigen Matrixschicht auf der Basis eines mit mehrwertigen Metallionen vernetzten, über freie Carboxylgruppen verfügenden Polyacrylatklebers, wobei die Matrix eine Fettsäure enthält, und
- einer vor Gebrauch zu entfernenden Schutzfolie,
**dadurch gekennzeichnet, daß** die wirkstoffhaltige Matrix einschichtig und frei von Hydroxylgruppen ist, und daß die Rückschicht aus
- einem elastischen Polyestergewebe oder Polyestergewirke oder
- einem vlies aus Polyethylenterephthalat, einem Gewebe aus Polyethylenterephthalat oder einem Gewirke aus Polyethylenterephthalat, oder
- einem geschlossenzelligen, elastischen Schaum besteht.

2. Topisches Pflaster gemäß Anspruch 1, dadurch gekennzaichnet, daß das nichtsteroidale Antirheumatikum ein Profenderivat ist.

3. Topischss Pflaster gemäß Anspruch 2, **dadurch gekennzeichnet, daß** der Wirkstoff Ketoprofen, Ibuprofen, Flurbiprofen oder Naproxen ist.

4. Topisches Pflaster gemäß Anspruch 3, **dadurch gekennzeichnet, daß** der Wirkstoff Ketoprofen oder Flurbiprofen ist und in einer Konzentration zwischen 5 und 15 Gew.% in der Pflastermatrix enthalten ist.

5. Topisches Pflaster gemäß Ansprach 4, **dadurch gekennzeichnet**, das der Wirkstoff Katoprofen ist und in einer Konsentration von 6-10 Gew.% in der Matrix gelöst vorliegt.

6. Topisches Pflaster gemäß Anspruch 1, **dadurch gekennzeichnet, daß** die Fettsäure Ölsäure, Linolsäure oder Linolensäure ist.

7. Topisches Pflaster gemäß Anspruch 6, **dadurch gekennzeichnet, daß** die Fettsäure Ölsäure ist und in einer Konzentration zwischen 5 und 20 Gew.% in der Pflastermatrix enthalten ist.

8. Topisches Pflaster gemäß Anspruch 1, **dadurch gekennzeichnet**, das der Polyacrylatkleber zumindest unter Verwendung von 2-Ethylhexylacrylat und Acrylsäure hergestellt wurde.

9. Topisches Pflaster gemäß Anspruch 1, **dadurch gekennzeichnet, daß** der Polyacrylatkleber unter Verwendung von 2-Ethylhexylacrylat, Vinylacetat, Acrylsäure, Butylacrylat hergestellt wurde.

10. Topisches Pflaster gemäß Anspruch 1, **dadurch gekennzeichnet, daß** die Rückschicht aus einem in zwei Richtungen elastischen Polyethylenterephthalatgewebe besteht.

11. Typisches Pflaster gemäß Anspruch 1, **dadurch gekennzeichnet, daß** der geschlossenzellige Schaum aus Polyethylen, Polypropylen, Polyvinylchlorid oder einem Copolymeren aus Ethylen und Vinylacetat besteht.

## Claims

1. Topical patch containing as active substance a non-steroid antirheumatic agent having a free carboxyl group, comprising
- a backing layer inert to the active substance, which backing layer is made of a material that is elastic in at least one direction,
- a self-adhesive, active substance-containing matrix layer based on a polyacrylate adhesive, which polyacrylate adhesive is crosslinked with multivalent metal ions and comprises free carboxyl groups, the said matrix containing a fatty acid, and
- a protective sheet be removed prior to use,
**characterized in that** said active substance-containing matrix consists of one layer and is free of hydroxyl groups, and that said backing layer is made of
- an elastic polyester woven fabric or polyester knitted fabric,
or
- a nonwoven of polyethylene terephthalate, a woven fabric of polyethylene terephthalate or a knitted fabric of polyethylene terephthalate, or
- a closed-cell, elastic foam.

2. Topical patch according to Claim 1, **characterized in that** the non-steroid antirheumatic agent is a profen derivative.

3. Topical patch according to Claim 2, **characterized in that** the active substance is ketoprofen, ibuprofen, flurprofen or naproxen.

4. Topical patch according to Claim 3, **characterized in that** the active substance is ketoprofen or flurprofen and is contained in the matrix of the patch in a concentration between 5 and 15%-wt.

5. Topical patch according to Claim 4, **characterized in that** the active substance is ketoprofen and is present, dissolved in the matrix, in a concentration of 6 - 10%-wt.

6. Topical patch according to Claim 1, **characterized in that** the fatty acid is oleic acid, linoleic acid or linolenic acid.

7. Topical patch according to Claim 6, **characterized in that** the fatty acid is oleic acid and is present in the matrix of the patch in a concentration between 5 and 20%-wt.

8. Topical patch according to Claim 1, **characterized in that** the polyacrylate adhesive has been produced using, at least, 2-ethylhexyl acrylate and acrylic acid.

9. Topical patch according to Claim 1, **characterized in that** the polyacrylate adhesive has been produced using 2-ethylhexyl acrylate, vinyl acetate, acrylic acid, butyl acrylate.

10. Topical patch according to Claim 1, **characterized in that** the backing layer consists of a polyethylene terephthalate woven fabric which is elastic in two directions.

11. Topical patch according to Claim 1, **characterized in that** the closed-cell foam consists of polyethylene, polypropylene, polyvinyl chloride or a copolymer of ethylene and vinyl acetate.

## Revendications

1. Emplâtre topique contenant, en tant que principe actif, un agent anti-rhumatismal non stéroïdien disposant d'un groupe carboxyle libre, constitué par
- une couche dorsale inerte vis-à-vis du principe actif, constituée d'un matériau qui est élastique suivant au moins une direction,
- une couche de matrice autocollante comprenant le principe actif, à base d'une colle de polyacrylate qui est réticulée avec des ions métalliques à valences multiples, disposant de groupes carboxyle libres, la matrice contenant un acide gras, et
- un film de protection à enlever avant usage,
**caractérisé en ce que** la matrice contenant le principe actif est constituée d'une seule couche et dépourvue de groupes hydroxyle, et **en ce que** la couche dorsale est constituée par
- un tissu ou tissu à mailles élastique en polyester ou
- une matière non-tissée en téréphtalate de polyéthylène, un tissu en téréphtalate de polyéthylène ou un tissu à mailles en téréphtalate de polyéthylène, ou
- une mousse élastique à cellules fermées.

2. Emplâtre topique selon la revendication 1, **caractérisé en ce que** l'agent anti-rhumatismal non stéroïdien est un dérivé de profène.

3. Emplâtre topique selon la revendication 2, **caractérisé en ce que** le principe actif est le kétoprofène, l'ibuprofène, le flurbiprofène ou le naproxène.

4. Emplâtre topique selon la revendication 3, **caractérisé en ce que** le principe actif est le kétoprofène ou le flurbiprofène et **en ce qu'**il est présent dans la matrice de l'emplâtre en une concentration comprise entre 5 et 15 % en poids.

5. Emplâtre topique selon la revendication 4, **caractérisé en ce que** le principe actif est le kétoprofène et **en ce qu'**il est présent en solution dans la matrice à une concentration comprise entre 6 et 10 % en poids.

6. Emplâtre topique selon la revendication 1, **caractérisé en ce que** l'acide gras est l'acide oléique, l'acide linoléique ou l'acide linolénique.

7. Emplâtre topique selon la revendication 6, **caractérisé en ce que** l'acide gras est l'acide oléique et **en ce qu'**il est présent dans la matrice d'emplâtre en une concentration comprise entre 5 et 20 % en poids.

8. Emplâtre topique selon la revendication 1, **caractérisé en ce que** la colle de polyacrylate est fabriquée en utilisant au moins l'acrylate de 2-éthylhexyle et l'acide acrylique.

9. Emplâtre topique selon la revendication 1, **caractérisé en ce que** la colle polyacrylate est fabriquée en utilisant au moins l'acrylate de 2-éthylhexyle, l'acétate de vinyle, l'acide acrylique et l'acrylate de butyle.

10. Emplâtre topique selon la revendication 1, **caractérisé en ce que** la couche dorsale est constituée d'un tissu en téréphtalate de polyéthylène qui est élastique suivant deux directions.

11. Emplâtre topique selon la revendication 1, **caractérisé en ce que** la mousse à cellules fermées est constituée de polyéthylène, de polypropylène, de poly(chlorure de vinyle) ou d'un copolymère d'éthylène et d'acétate de vinyle.
